# EUROPÄISCHE PATENTANMELDUNG

(11) **EP 1 733 727 A1**
(43) Veröffentlichungstag der Anmeldung: **20.12.2006**
(21) Anmeldenummer: 06020274.4
(22) Anmeldetag: 02.07.2003
(51) Int. Cl.: A61K 31/496, A61P 25/00, C07F 17/02

(54) **Ferrocencarboxamide zur Verwendung als Dopamin D3-Liganden zur Behandlung von ZNS-Erkrankungen**

(30) Priorität: 04.07.2002 DE 10230062; 10.07.2002 DE 10232020
(62) Teilanmeldung aus: 03762588.6
(71) Anmelder: SCHWARZ PHARMA AG, 40789 Monheim/Rhld. (DE)
(72) Erfinder: Gmeiner, Peter, 91054 Erlangen-Buckenhof (DE); Hübner, Harald, 91336 Heroldsbach (DE); Schlotter, Karin, 86732 Oettingen (DE)
(74) Vertreter: HOFFMANN EITLE

(57) **Zusammenfassung**

Die vorliegende Erfindung betrifft neurorezeptoraktive N-[(4-Phenyl-1-piperazinyl)alkyl]-substituierte 2-Ferrocenylverbindungen der allgemeinen Formel (II) und ihre Verwendung zur Behandlung von ZNS-Erkrankungen, wie beispielsweise Schizophrenie, verschiedene Arten der Depression, neurodegenerative Störungen, sexuelle Dysfunktionen, Kokain-, Alkohol-, Opiat- und Nikotinsucht, sowie von Glaukoma, kognitiven Störungen, Restless Leg Syndrom, Hyperaktivitätssyndrom (ADHS), Hyperprolaktinämie, Hyperprolaktinom, Parkinson-assoziierten Bewegungsstörungen, Behandlung von L-DOPA und Neuroleptika-induzierten Bewegungsstörungen, z.B. Akathisie, Rigor, Dystonie und Dyskinesien: 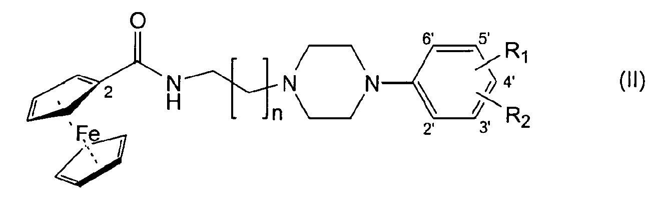 worin die Substituenten wie in der Beschreibung definiert sind.

## Beschreibung

Dopamin gilt als wichtiger Neurotransmitter des zentralen Nervensystems. Seine Wirkung vermittelt Dopamin durch Bindung an fünf verschiedenen Dopaminrezeptoren. Diese lassen sich aufgrund ihrer Morphologie und ihrer Art der Signalübertragung in die Klassen D1-like (D1 und D5) sowie D2-like (D2-, D3- und D4-Rezeptoren) einteilen (Neve, K.A. The Dopamine Receptors. Humana Press, 1997). Vor allem die Subtypen der D2-Familie spielen bei der Regulation zentralnervöser Vorgänge eine wichtige Rolle. Während die D2-Rezeptoren überwiegend in den Basalganglien exprimiert werden und dort neuromotorische Schaltkreise kontrollieren, finden sich D3-Rezeptoren vor allem im limbischen System, in dem emotionale und kognitive Vorgänge gesteuert werden. Störungen in der Signaltransduktion dieser Rezeptoren führen zu zahlreichen neuropathologischen Situationen. Insbesondere der D3-Rezeptor gilt als vielversprechendes Target für die Entwicklung von Wirkstoffen zur Behandlung von psychiatrischen Erkrankungen wie der Schizophrenie oder der unipolaren Depressionen, von Bewusstseinsstörungen sowie zur Behandlung neurodegenerativer Krankheiten wie dem Parkinsonismus, aber auch zur Behandlung von Drogenabhängigkeit (Pulvirenti, L. et al. Trends Pharmacol. Sci. 2002, 23, 151-153).

Verbindungen mit Arylpiperazin-Struktur sind bereits als dopaminrezeptoraktive Liganden beschrieben worden (Robarge, M.J. J. Med. Chem. 2001, 44, 3175-3186). Weiterhin sind Benzamide und Naphthamide mit Arylpiperazin-Partialstruktur als Liganden von Dopaminrezeptoren bekannt (Perrone, R. J. Med. Chem. 1998, 41, 4903-4909; EP 0 779 284 A1). Kürzlich wurde ein Phenylpiperazinyinaphthamid als selektiver D3-Partialagonist beschrieben, der im Tiermodell hoffnungsvolle Aktivitäten zeigt, die für die Behandlung der Kokainsucht eingesetzt werden könnten (Pilla, M. et al. Nature 1999, 400, 371-375).

Für wenige Beispiele wurden bisher Arylpiperazinylamide mit sauerstoff-, schwefel- oder stickstoffhaltigen Heteroarensäurekomponenten beschrieben (ES 2027898; EP 343 961; US 3646047; US 3734915). Demgegenüber sind cyanosubstituierte sowie tellurhaltige Derivate und Verbindungen mit Ferrocenylpartialstruktur in der Literatur nicht bekannt.

Wir haben im Rahmen unserer Struktur-Wirkungsuntersuchungen von Dopaminrezeptorliganden neue Verbindungen der Formel (11) entdeckt. Diese zeigten bei *in vitro* Untersuchungen hochaffine und hochselektive Bindungseigenschaften am D3-Rezeptor, wie sie bislang noch nicht bekannt sind. Die Verbindungen könnten somit wertvolle Therapeutika zur Behandlung von ZNS-Erkrankungen, wie beispielsweise Schizophrenie, verschiedene Arten der Depression, neurodegenerative Störungen, sexuelle Dysfunktionen sowie der Kokain-, Alkohol-, Opiat- und Nikotinsucht darstellen.

Weiterhin sollten als konkrete Einsatzmöglichkeiten genannt werden: Glaukoma, kognitive Störungen, Restless Leg Syndrom, Hyperaktivitätssyndrom (ADHS), Hyperprolaktinämie, Hyperprolaktinom, Parkinson-assozüerte Bewegungsstörungen, Behandlung von L-DOPA und Neuroleptika induzierten Bewegungsstörungen, z.B. Akathisie, Rigor, Dystonie und Dyskinesien.

Gegenstand dieser Erfindung sind Derivate von 2-Heteroarencarbonsäureamiden mit Arylpiperazinylpartialstruktur in Form der freien Base und Salze davon, wie sie durch die folgenden Formel (11) wiedergegeben sind:

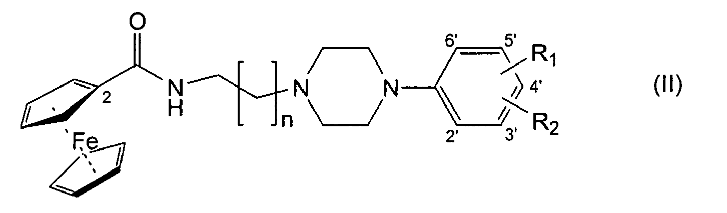

In Formel (II) stehen für:
n = 1 - 4 sowie R₁ und R₂ einzeln oder auch zusammen für die Reste Wasserstoff, Hydroxy, Alkyloxy, Alkyl, Alkenyl, Alkinyl, Aryl, Halogen, Trifluormethyl, Acyl, Alkoxycarbonyl oder Cyano.

Die Erfindung betrifft insbesondere physiologisch akzeptable Salze der erfindungsgemäßen Verbindungen.

Dem Fachmann ist ferner klar, dass je nach Wahl der Substituenten optisch aktive Verbindungen entstehen können. In diesem Fall sind sowohl die Racemate als auch die jeweiligen reinen enantiomeren Formen Gegenstand der vorliegenden Erfindung.

Die in der Beschreibung und in den anliegenden Patentansprüchen genannten Substituenten umfassen insbesondere die nachfolgend erläuterten Gruppen.

"Alkyl" kann eine verzweigte oder unverzweigte Alkylgruppe sein, die vorzugsweise 1 bis 10 C-Atome, besonders bevorzugt 1 bis 6 C-Atome und ganz besonders bevorzugt 1, 2 oder 3 C-Atome aufweist, z.B. Methyl, Ethyl, n-Propyl, iso-Propyl, n-Butyl, iso-Butyl, s-Butyl, t-Butyl, n-Pentyl, iso-Pentyl, Neopentyl, t-Pentyl, 1-Methylbutyl, 2-Methylbutyl, 1-Ethylpropyl, 1,2-Dimethylpropyl und n-Hexyl. Alkylgruppen können zusätzlich mit einem oder mehreren Substituenten substituiert sein, beispielsweise mit Halogen oder einer oder mehreren Phenylgruppen.

"Alkenyl" und "Alkinyl" weisen mindestens eine Doppel- bzw. Dreifachbindung auf. Sie können verzweigt oder unverzweigt sein und weisen vorzugsweise 2 bis 6 C-Atome auf. Alkenyle oder Alkinyle sind vorzugsweise so an den Heteroaren- oder Phenylring des Grundgerüsts der Verbindung gebunden, dass die Doppel- bzw. Dreifachbindung mit dem aromatischen Ring konjugiert ist. Alkenyl und Alkinyl können zusätzlich mit einem oder mehreren Substituenten substituiert sein, vorzugsweise mit Phenyl, wobei sich die Phenylgruppe dann besonders bevorzugt am C-Atom 2 befindet (wenn Alkenyl oder Alkinyl über das C-Atom 1 an den Heteroaren- oder Phenylring des Grundgerüsts gebunden ist). Bevorzugt sind die Alkenyle oder Alkinyle unsubstituiert.

"Alkyloxy" ist die Gruppe -O-Alkyl, worin Alkyl vorzugsweise aus den oben für "Alkyl" angegebenen Gruppen ausgewählt ist. Bevorzugt ist Alkyloxy eine C1-C6-Alkyloxygruppe, speziell Methoxy. In einer anderen Ausführungsform kann Alkyloxy auch eine C2-C6-Alkyloxygruppe sein.

"Aryl" ist bevorzugt Phenyl. Phenyl kann gegebenenfalls zusätzlich in einer oder mehreren der Positionen 2, 3 und 4 unabhängig substituiert sein, z.B. mit Alkoxy, Trifluormethyl oder Halogen, bevorzugt mit Methoxy.

"Acyl" umfasst insbesondere die Gruppen -C(O)-Alkyl und -C(O)-Aryl, worin Alkyl und Aryl vorzugsweise aus den oben für "Alkyl" bzw. "Aryl" angegebenen Gruppen ausgewählt sind, insbesondere -C(O)-C1-C6-Alkyl. Acyl ist beispielsweise Acetyl, Propionyl, Butyryl oder -C(O)-Phenyl.

"Alkoxycarbonyl" ist die Gruppe -C(O)-O-Alkyl, worin Alkyl vorzugsweise aus den oben für "Alkyl" angegebenen Gruppen ausgewählt ist. Bevorzugt ist Alkoxycarbonyl eine (C1-C6-Alkyl)oxycarbonylgruppe.

"Halogen" ist bevorzugt Fluor, Chlor, Brom oder lod.

"Physiologisch akzeptable Salze" schließen nicht-toxische Additionssalze einer Base mit organischen oder anorganischen Säuren ein. Beispiele für anorganische Säuren schließen HCl, HBr, Schwefelsäure und Phosphorsäure ein. Organische Säuren schließen Essigsäure, Propionsäure, Brenztraubensäure, Buttersäure, α-, β- oder γ-Hydroxybuttersäure, Valeriansäure, Hydroxyvaleriansäure, Capronsäure, Hydroxycapronsäure, Caprylsäure, Caprinsäure, Laurinsäure, Myristinsäure, Palmitinsäure, Stearinsäure, Glykolsäure, Milchsäure, D-Glucuronsäure, L-Glucuronsäure, D-Galacturonsäure, Glycin, Benzoesäure, Hydroxybenzoesäure, Gallussäure, Salicylsäure, Vanillinsäure, Cumarsäure, Kaffeesäure, Hippursäure, Orotsäure, L-Weinsäure, D-Weinsäure, D,L-Weinsäure, meso-Weinsäure, Fumarsäure, L-Äpfelsäure, D-Äpfelsäure, D,L-Äpfelsäure, Oxalsäure, Malonsäure, Bernsteinsäure, Maleinsäure, Oxalessigsäure, Glutarsäure, Hydroxyglutarsäure, Ketoglutarsäure, Adipinsäure, Ketoadipinsäure, Pimelinsäure, Glutaminsäure, Asparaginsäure, Phthalsäure, Propantricarbonsäure, Zitronensäure, Isozitronensäure, Methansulfonsäure, Toluolsulfonsäure und Trifluormethansulfonsäure ein.

Bevorzugte Verbindung der allgemeinen Formel (II) wie oben definiert sind solche, in denen R₁ und R₂ jeweils unabhängig voneinander ausgewählt sind aus Wasserstoff, Hydroxy, C1-C6-Alkyloxy, C1-C6-Alkyl, C2-C6-Alkenyl, C2-6-Alkinyl, Aryl, Fluor, Chlor, Brom, Trifluormethyl, C1-C6-Acyl, C1-C6-Alkoxycarbonyl und Cyano.

Konkrete Verbindungen der allgemeinen Formel (II) sind
(B2): N-4-(4-(2-Methoxyphenyl)piperazin-1-yl)butyl-2-ferrocenylcarbamid und
(B3): N-4-(4-(2,3-Dichlorphenyl)piperazin-1-yl)butyl-2-ferrocenylcarbamid

Die erfindungsgemäßen Verbindungen der Formel (II), wie definiert, sind zum therapeutischen Einsatz als Dopamin D3-Liganden geeignet.

Der Begriff "hochaffine D3-Liganden" umfasst Verbindungen, die in einem Radioligandexperiment Bindung an humane Dopamin D3-Rezeptoren mit einem Ki-Wert von vorzugsweise nicht mehr als 10 nM, besonders bevorzugt nicht mehr als 1 nM zeigen (vgl. Hübner, H. et al. J. Med. Chem. 2000, 43, 756-762 sowie nachfolgender Abschnitt "Biologische Aktivität").

Ein Aspekt der vorliegenden Erfindung betrifft selektive D3-Liganden. Der Begriff "selektive D3-Liganden" umfasst Verbindungen, die im Radioligandexperiment für den D3-Rezeptor, wie im nachfolgenden Abschnitt "Biologische Aktivität" beschrieben, einen Ki-Wert aufweisen, der um einen Faktor von zumindest 10 niedriger als für mindestens fünf der sieben folgenden Rezeptoren ist: Dopamin-Rezeptoren D1, D2long, D2short und D4.4, Serotonin-Rezeptoren 5-HT1A und 5-HT2 und Alpha 1 Adrenozeptor.

Ein anderer Aspekt der Erfindung betrifft hochselektive Dopamin D3-Liganden. Der Begriff "hochselektive D3-Liganden" umfasst Verbindungen, die im Radioligandexperiment für den D3-Rezeptor, wie im nachfolgenden Abschnitt "Biologische Aktivität" beschrieben, einen Ki-Wert aufweisen, der um einen Faktor von zumindest 100 niedriger als für mindestens drei, bevorzugt für alle der Dopamin-Rezeptoren D1, D2long, D2short und D4.4 ist.

D3-Liganden können am D3-Rezeptor agonistische, antagonistische oder partialagonistische Wirkung haben. Die entsprechenden intrinsischen Aktivitäten der erfindungsgemäßen Verbindungen lassen sich in Mitogeneseassays messen, wie in der Literatur beschrieben (Hübner, H. et al. J. Med. Chem. 2000, 43, 4563-4569 und Löber, S. Bioorg. Med. Chem. Lett. 2002, 12.17, 2377-2380). In Abhängigkeit von der Pathophysiologie der zugrunde liegenden Erkrankung kann therapeutisch eine stärker agonistische, stärker antagonistische oder eine partialagonistische Aktivität gewünscht sein. Die vorliegende Erfindung erlaubt daher in exzellenter Weise eine Feineinstellung der gewünschten Aktivität.

Ein weiterer Gegenstand der Erfindung ist daher ein Arzneimittel, das eine oder mehrere der Verbindungen der allgemeinen Formel (II) oder eine der konkret aufgeführten Verbindungen wie oben definiert, gegebenenfalls in Form eines pharmazeutisch akzeptablen Salzes, enthält.

Die Erfindung betrifft ebenfalls die Verwendung einer oder mehrerer der Verbindungen der allgemeinen Formel (II) oder einer der konkret aufgeführten Verbindungen, gegebenenfalls in Form eines pharmazeutisch akzeptablen Salzes, zur Behandlung, einschließlich Therapie und Prophylaxe, der hier genannten Indikationen sowie zur Herstellung eines Arzneimittels für die hier genannten Indikationen.

Bevorzugt werden zur Herstellung von Arzneimitteln solche erfindungsgemäßen Verbindungen ausgewählt, die selektive D3-Liganden sind. Besonders bevorzugt werden hochselektive D3-Liganden verwendet.

Die erfindungsgemäßen Verbindungen haben Potential in der Therapie oder Prophylaxe einer Reihe von Erkrankungen, die insbesondere mit einer Störung des Dopaminstoffwechsels oder der dopaminergen Signalkaskade einhergehen.

Beispiele für solche Erkrankungen sind Kokain-, Alkohol-, Opiat- und Nikotinsucht; neurodegenerative Störungen, insbesondere Morbus Parkinson; sexuelle Dysfunktion, insbesondere männliche erektile Dysfunktion; Depression, insbesondere endogene monophasische Depression ("major depression") und Schizophrenie.

Weitere Beispiele, die der Therapie oder Prophylaxe mit den erfindungsgemäßen Verbindungen zugänglich sind, sind Hyperprolaktinämie; Hyperprolaktinom; Glaucoma; kognitive Störungen; Restless Leg Syndrom; Hyperaktivitätssyndrom (ADHS); Parkinsonassoziierte Bewegungsstörungen, z.B. Rigor, Dystonie und Dyskinesie; L-Dopa-induzierte Störungen, z.B. Angstzustände, Schlafstörungen, Psychosen, Dyskinesien und Dystonien; idiopathische Dystonien, insbesondere Segawa-Syndrom; Neuroleptika-induzierte (tardive) Dyskinesie, Dystonie und Akathisie.

Insbesondere sind die erfindungsgemäßen Verbindungen zur Herstellung eines Arzneimittels zur Behandlung Dopa-sensitiver Bewegungsstörungen geeignet. Solche Bewegungsstörungen können beispielsweise Dyskinesien, Dystonien, Rigor und Tremor sein. Unter dem Begriff "Dopa-sensitiv" wird dabei verstanden, dass die Bewegungsstörung günstig durch Gabe von Arzneimitteln beeinflusst werden kann, die die dopaminerge Signalübertragung beeinflussen. Ein typisches Beispiel hierfür ist das Segawa-Syndrom, eine idiopathische Dystonie, bei der das Ansprechen auf L-Dopa als diagnostisches Kriterium genutzt werden kann.

Eine bevorzugte Verwendung betrifft die Herstellung eines Arzneimittels zur Behandlung von Dyskinesien und Dystonien, die spontan im Zuge von Parkinson-Erkrankungen auftreten können, aber auch Medikamenten-induziert sein können. Unter den Medikamenten-induzierten Dyskinesien und Dystonien sind insbesondere solche zu nennen, die durch Neuroleptika bzw. Dopaminantagonisten oder Dopaminagonisten oder L-Dopa induziert wurden.

Ferner können die Arzneimittel zum Medikamenten-unterstützten Abstillen nach Schwangerschaften eingesetzt werden.

Schließlich können die erfindungsgemäßen Arzneimittel in Abhängigkeit von der zu behandelnden Erkrankung auch als Kombinationspräparat zur gleichzeitigen oder sequentiellen Gabe ausgebildet sein.

Beispielsweise kann eine Verkaufseinheit, die eine zur Behandlung der Parkinson-Erkrankung enthaltende L-Dopa Medikation enthält, auch eine pharmazeutische Zusammensetzung umfassen, die eine der erfindungsgemäßen Verbindungen mit z.B. hochselektivem, partial-agonistischem Wirkprofil enthält. Dabei können L-Dopa und die erfindungsgemäße Verbindung in der gleichen pharmazeutischen Formulierung, z.B. einer Kombinationstablette, oder auch in unterschiedlichen Applikationseinheiten vorliegen, z.B. in Form zweier separater Tabletten. Je nach Bedarf können beide Wirkstoffe gleichzeitig oder zeitlich getrennt verabreicht werden.

In einem Kombinationspräparat kann eine sequentielle Gabe beispielsweise erreicht werden, indem eine Darreichungsform, z.B. eine orale Tablette, zwei unterschiedliche Schichten mit differierendem Freisetzungsprofil für die verschiedenen pharmazeutisch aktiven Bestandteile aufweist. Dem Fachmann ist klar, dass im Kontext der vorliegenden Erfindung verschiedene Darreichungsformen und Applikationsschemata denkbar sind, die alle Gegenstand der Erfindung sind.

Eine Ausführungsform der Erfindung betrifft daher ein Arzneimittel, das L-Dopa oder ein Neuroleptikum sowie eine erfindungsgemäße Verbindung zur gleichzeitigen oder zeitlich aufeinanderfolgenden Verabreichung an den Patienten enthält.

Üblicherweise bestehen die erfindungsgemäßen Arzneimittel aus einer pharmazeutischen Zusammensetzung, die neben den erfindungsgemäßen D3-Liganden, wie oben beschrieben, mindestens einen pharmazeutisch annehmbaren Träger oder Hilfsstoff enthält.

Dem Fachmann ist klar, dass die pharmazeutische Formulierung in Abhängigkeit vom beabsichtigten Applikationsweg unterschiedlich ausgestaltet sein kann. So kann die pharmazeutische Formulierung beispielsweise zur intravenösen, intramuskulären, intrakutanen, subkutanen, oralen, bukkalen, sublingualen, nasalen, transdermalen, inhalativen, rektalen oder intraperitonealen Verabreichung angepasst sein.

Entsprechende Formulierungen und hierfür geeignete pharmazeutische Träger bzw. Hilfsstoffe, wie Füllstoffe, Sprengmittel, Bindemittel, Gleitmittel, Stabilisatoren, Aromastoffe, Antioxidantien, Konservierungsmittel, Dispersions- oder Lösungsmittel, Puffer oder Elektrolyte, sind dem Fachmann auf dem Gebiet der Pharmazeutik bekannt und sind beispielsweise in Standardwerken wie Sucker, Fuchs und Speiser ("Pharmazeutische Technologie", Deutscher Apotheker Verlag, 1991) und Remington ("The Science and Practice of Pharmacy", Lippincott, Williams & Wilkins, 2000) beschrieben.

In einer bevorzugten Ausführungsform der Erfindung werden die pharmazeutischen Zusammensetzungen, die die erfindungsgemäßen Verbindungen enthalten, oral verabreicht und können beispielsweise als Kapsel, Tablette, Pulver, Granulat, Dragee oder in flüssiger Form vorliegen.

Dabei kann die Formulierung als schnell freisetzende Darreichungsform ausgestaltet sein, wenn ein rascher Wirkeintritt gewünscht ist. Entsprechende orale Formulierungen sind beispielsweise beschrieben in EP 0 548 356 oder EP 1 126 821.

Ist dagegen eine protrahierte Freisetzung erwünscht, bietet sich eine Formulierung mit retardierter Wirkstofffreisetzung an. Entsprechende orale Formulierungen sind ebenfalls aus dem Stand der Technik bekannt.

Alternative pharmazeutische Zubereitungen können beispielsweise Infusions- oder Injektionslösungen, Öle, Suppositorien, Aerosole, Sprays, Pflaster, Mikrokapseln oder Mikropartikel sein.

Die Verbindungen wurden nach den Methoden entsprechend der Literatur (Glennon, R.A et al. J. Med. Chem. 1988, 31, 1968-1971) hergestellt.

Alternativ zur oben erwähnten Methode der Aktivierung der Säurederivate können auch andere Reaktionen eingesetzt werden, wie zum Beispiel die Aktivierung von Säuren durch Hydroxyazabenzotriazol (Kienhöfer, A. Synlett 2001, 1811-1812). Beispielsweise können die Verbindungen der Formel (II) durch Aktivierung von Ferrocen-2-carbonsäure mit HATU und anschließende Reaktion mit den Basen des Typs (B) hergestellt werden.

Für die Herstellung der Arylpiperazinylamine vom Typ (B) können z.B. käuflich zugängliche 2-Methoxy- bzw. 2,3-Dichlorphenylpiperazine mit Brombutylphthalimid in Xylol alkyliert werden. Anschließende Hydrazinolyse der phthalimidsubstituierten Strukturen liefert die primären Amine vom Typ (B). Dies wird anhand des folgenden exemplarischen Reaktionsschemas verdeutlicht:

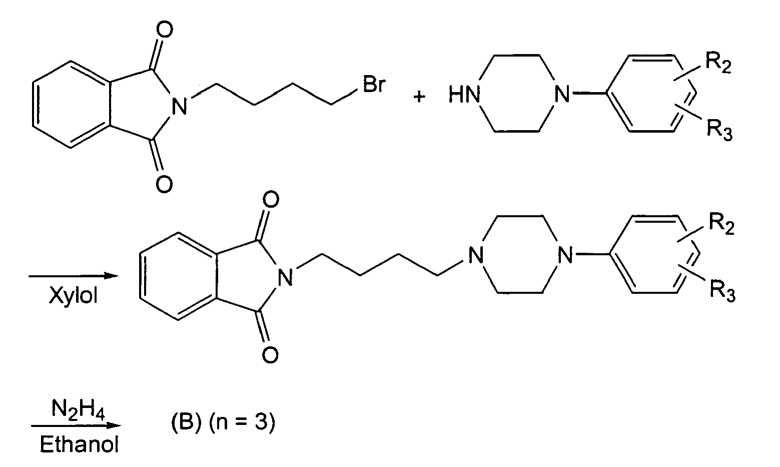

### BEISPIELE

### Synthese der Amine vom Typ (B)

### 4-(4-(2, 3-Dichlorphenyl)piperazin-1-yl)butylamin

2,3 g (10 mmol) freie Base des 2,3-Dichlorphenylpiperazins werden in 10 ml Xylol gelöst und auf 70°C erhitzt. Dann werden 1,4 g (5 mmol) 4- Brombutylphthalimid in 20 ml Xylol gelöst and langsam der Lösung des Piperazins zugetropft. Das Reaktionsgemisch wird für 24 Stunden bei 125°C erhitzt. Nach Abkühlen der Mischung auf 0°C wird abfiltriert and das Filtrat evaporiert. Das entstandene N-4-(4-(2,3-Dichlorphenyl)piperazin-1-yl)butylphthalimid wird durch Flashchromatographie an SiO₂ mit Ethylacetat gereinigt. Ausbeute: 4,0 g (= 92%)

Zu einer Suspension von N-4-(4-(2,3-Dichlorphenyl)piperazin-1-yl)butylphthalimid in 40 ml Ethanol wird eine Lösung von 80% Hydrazinhydrat (0,45 ml, 2,5 eq) in 5 ml Ethanol zugetropft. Die Mischung wird für 3 Stunden unter Rückfluss erhitzt, anschließend auf RT abgekühlt, der dabei ausfallende Feststoff abfiltriert, und die ethanolische Lösung im Vakuum abgedampft.

Reinigung durch Flashchromatographie mit CH₂Cl₂-MeOH-Me₂EtN:90-8-2 liefert die Base 4-(4-(2,3-Dichlorphenyl)piperazin-1-yl)butylamin mit einer Ausbeute von 900 mg (= 60 %).

MS: m/z 301 (M+); IR: (NaCl): 3397; 2939; 2817; 1641; 1572; 1500; 1482; 1452; 1376; 1240; 1152; 1118; 1023; 917; 791; 749; 698; 661. 1 H-NMR (CDCl₃, 360 MHz) δ (ppm): 1,48-1,64 (m, 4H,CH₂-CH₂);2,41-2,46 (t, J = 7,6, 2H, CH₂N); 2,64 (m, 4H, pip); 2,72-2,76 (m, 2H, CH₂NCO); 3,07 (m, 4H, pip); 6,93-6,99 (m, 1 H, H₅, Phenyl); 7,11-7,17 (m, 2H, H₄, H₆, Phenyl).

### Beispiel 1 (Referenz)

### N-4-(4-(2-Methoxyphenyl)piperazin-1-yl)butyl-5-cyan-2-benzo[b]furanylcarbamid

0,37 mmol (141 mg) HATU und 0,37 mmol (69 mg) der 5-Cyano-2-benzo[b]furanylcarbonsäure (Dann, O. Liebigs Ann. Chem. 1986, 438-455) werden bei 0°C in 1 ml DMF gelöst und 0,74 mmol (0,13 ml) DIEA zugegeben. Anschließend wird 0,33 mmol (87 mg) 4-(4-(2-Methoxyphenyl)piperazin-1-yl)butylamin in DMF gelöst and bei 0°C zu der Reaktionslösung zugetropft. Nach 1 Stunde wird der Reaktionsansatz in CHCl₃ aufgenommen und mit NaHCO₃-Lösung sowie Wasser gewaschen. Nach Trocknung mit MgSO₄ wird das Lösungsmittel abgedampft und durch Flashchromatographie (SiO₂; Petrolether-Ethylacetat: von 1-1 nach Ethylacetat) gereinigt.
Ausbeute: 41 mg (28%)

Smp.: 96°C. MS m/z 432 (M⁺). IR (NaCl): 3400; 2937; 2815; 2227; 1666; 1594; 1527; 1500; 1294; 1240; 1141; 1118; 1025; 842; 746. ¹H-NMR (CDCl₃, 360 Mhz) δ (ppm): 1,67-1,74 (m, 4H, CH₂-CH₂); 2,49 (t, J=6,9 Hz, 2H, CH₂N); 2,69 (m, 4H, pip); 3,13 (m, 4H, pip); 3,56-3,60 (m, 2H, CH₂NCO); 3,86 (s, 3H, OCH₃); 6,84-7,02 (m, 4H, Phenyl); 7,12-7,15 (brt, J=5,1 Hz, 1H, NH); 7,50-7,51 (m, 1 H, H₄); 7,55-7,57 (m, 1 H, H₃); 7,65-7,68 (m ,1H, H₆); 8,03-8,04 (m, 1H, H₇). ¹³C-NMR (CDCl₃, 90 MHz) δ (ppm): 157,9; 156,1; 152,3; 151,1; 141,2; 129,9; 127,8; 123,0; 121,0; 118,2; 113,0; 112,3; 106,6; 109,7; 107,9; 57,9; 55,4; 53,5; 50,5; 39,4; 27,4; 24,3; 21,0.

### Beispiel 2

### N-4-(4-(2-Methoxyphenyl)piperazin-1-yl)butyl-2-ferrocenylcarbamid

Synthese analog zu Beispiel 1.
Ausbeute: 125 mg (71%)

MS m/z475 (M⁺). IR (NaCl): 3318, 2939, 2816, 1630, 1543, 1500, 1241, 1027, 748. ¹H-NMR (CDCl₃, 360 MHz) δ (ppm): 1,66-1,69 (m, 4H, CH₂-CH₂); 2,57 (t, J=6,7 Hz, 2H, CH₂N); 2,77 (m, 4H, pip); 3,16 (m, 4H, pip); 3,39-3,44 (m, 2H, CH₂NCO); 3,86 (s, 3H, OCH₃); 4,17-4,21 (m, 3H, Ferr); 4,32-4,33 (m, 2H, Ferr); 4,68-4,69 (m, 2H, Ferr); 6,02 (brt, J=5,3 Hz, 1 H, NH); 6,8 5-6,94 (m, 3H, Phenyl); 6,99-7,03 (m, 1 H, Phenyl). ¹³C-NMR (CDCl₃, 90 MHz) δ (ppm): 170,3; 152,2; 140,9; 123,1; 120,9; 118,3; 111,2; 77,2; 76,3; 70,3; 69,7; 68,1; 57,9; 55,3; 53,4; 50,1; 39,2; 27,7; 23,8.

### Beispiel 3

### N-4-(4-(2,3-Dichlorphenyl)piperazin-1-yl)butyl-2-ferrocenylcarbamid

Synthese analog zu Beispiel 1.
Ausbeute: 85 mg (69 %)

MS m/z 514 (M⁺). ¹H-NMR (CDCl₃, 360 MHz) δ (ppm): 1,63-1,66 (m, 4H, CH₂-CH₂); 2,48 (t, J=6,9 Hz, 2H, CH₂N); 2,65 (m, 4H, pip); 3,08 (m, 4H, pip); 3,39-3,44 (m, 2H, CH₂NCO); 4,17-4,21 (m, 3H, Ferr); 4,33-4,34 (m, 2H, Ferr); 4,64-4,6 5 (m, 2H, Ferr); 5,38 (brt, J=5,1 Hz, 1H, NH); 6,92-6,98 (m, 1H, Phenyl); 7,11-7,17 (m, 2H, Phenyl).

### BIOLOGISCHE AKTIVITÄT

Die biologischen Aktivitäten der erfindungsgemäßen Verbindungen wurden in Radioligandbindungsuntersuchungen ermittelt. Alle Radioligandexperimente wurden nach von uns beschriebenen Methoden durchgeführt (Hübner, H. et al. J. Med. Chem. 2000, 43, 756-762). Für die Messung der Affinitäten zu den Rezeptoren der D2-Familie kamen Membranhomogenate von chinesischen Hamster-Ovarialzellen (CHO-Zellen) zum Einsatz, die jeweils den humanen D2long-, den humanen D2short- (Hayes, G. et al. Mol. Endocrinol. 1992, 6, 920-926), den humanen D3- (Sokoloff, P. et al. Eur. J. Pharmacol. 1992, 225, 331-337) oder den humanen D4.4-Rezeptorsubtyp (Asghari, V. J. Neurochem. 1995, 65, 1157-1165) stabil exprimierten. Prinzipiell erfolgten die Bindungsassays durch Inkubation der Rezeptorhomogenate mit dem Radioligand [³H]Spiperon und der zu untersuchenden Verbindung in verschiedenen Konzentrationen. Die Ermittlung der Affinitäten zum D1-Rezeptor erfolgte mit nativen Membranhomogenaten, gewonnen aus dem Striatum des Schweines, und dem D1-selektiven Radioliganden [³H]SCH 23390.

Zur Bestimmung der Bindungsstärken der Verbindungen zu den SerotoninRezeptorsubtypen 5-HT1A und 5-HT2 wurden Cortex-Membranpräparationen des Schweines mit den Radioliganden [³H]8-OH-DPAT (für 5-HT1A) oder [³H]Ketanserin (5-HT2) und den Verbindungen inkubiert. Hinweise auf eine gleichzeitige Bindung der Verbindungen an den serotonergen 5-HT2-Rezeptor und an den adrenergen Rezeptorsubtyp α1 bei der Markierung mit dem Radioliganden [³H]Ketanserin wurden in einem Parallelexperiment bei selektiver Blockade des α1-Rezeptors durch Prazosin bestätigt. Somit repräsentieren Ki-Werte, die in Anwesenheit von 10 µM Prazosin ermittelt wurden, die alleinige Bindung an den 5-HT2-Rezeptor. Ergänzend wurde die Affinität zu α1-Rezeptoren des Schweines in einem separaten Experiment mit dem α1-selektiven Radioliganden [³H]Prazosin bestimmt.

Die Ergebnisse der Rezeptorbindungsuntersuchungen an den Dopaminrezeptorsubtypen sind in Tabelle 1 zusammengefasst.

Alle im Bindungsassay untersuchten Verbindungen zeigten dabei gute bis sehr gute Affinitäten zu den Dopaminrezeptoren mit einer klaren Bindungspräferenz zu den Subtypen der D2-Familie. Unabhängig von der Partialstruktur ist dabei immer eine deutliche Selektivität zum D3-Rezeptor zu erkennen.

Das Substitutionsmuster der Arylpiperazinylkomponente beeinflusst vor allem die Ausprägung der Selektivität der D3-Affinität gegenüber den anderen Rezeptorsubtypen. Interessanterweise sind die Ferrocenylderivate der Beispiele 2 und 3 durch eine hohe D4-Affinität charakterisiert, wobei Beispiel 3 mit Ki-Werten von 0,47 nM für den D3-Rezeptor und 0,63 nM für den D4-Rezeptor ein sehr außergewöhnliches Rezeptorbindungsprofil aufweist.

Untersuchungen zur Bestimmung der intrinsischen Aktivität der Beispielverbindungen wurden in einem Mitogeneseassay in Anlehnung an die Literatur (Hübner, H. et al. J. Med. Chem. 2000, 43, 4563-4569; Löber, S. Bioorg. Med. Chem. Lett. 2002, 12.17, 2377-2380) durchgeführt.

**Tabelle 1: Bindungsdaten und Selektivitätsmuster der Verbindungen für die Dopamin-Rezeptoren pD1, hD2long, hD2short, hD3 und hD4.4**

| Verbindung | Ki-Werte in [nM]^{a} | | | | | D3-Selektivität | | |
|---|---|---|---|---|---|---|---|---|
| | D1 | D2long | D2short | D3 | D4.4 | D2long/D3 | D2short/D3 | D4.4/D3 |
| **Beispiel 2** | 1500 | 110 | 78 | 6,5 | 0,40 | 17 | 12 | 0,061 |
| **Beispiel 3** | 630 | 31 | 19 | 0,47 | 0,63 | 66 | 40 | 1,3 |

| | | | | | | | | |
|---|---|---|---|---|---|---|---|---|
| ^{a} Durchschnittswerte aus 2-9 Einzelexperimenten durchgeführt als Triplikate | | | | | | | | |

Die Untersuchung der Affinitäten zu den Serotoninrezeptorsubtypen 5-HT1A und 5-HT2 sowie zum adrenergen Rezeptor α1 sind in Tabelle 2 beschrieben. Dabei ist unabhängig von den Partialstrukturen der Derivate eine bevorzugte Bindung an den 5-HT1A-Subtyp im Vergleich zu 5-HT2 zu erkennen. Die Verbindung 2 ist mit einem gemessenen Ki-Wert von 19 nM durch eine hohe Affinität zum α1-Rezeptor charakterisiert.

Struktur-Wirkungs-Überlegungen zeigen für die Bindung an diese Rezeptoren eine deutliche Abhängigkeit vom Substitutionsmuster der Arylpiperazinylpartialstruktur. Wie bei den Dopaminrezeptoren geht bei den Derivaten mit 2,3-Dichlorphenylrest die Bindung zu den 5-HT- und zum α1-Rezeptor deutlich zurück, was zu einer Erweiterung des Selektivitätsspektrums gegenüber der D3-Rezeptoraffinität dieser Verbindungen führt.

**Tabelle 2: Bindungsdaten der Substanzen für die Serotonin-Rezeptoren p5-HT1A, p5-HT2 sowie für den adrenergen Rezeptorsubtyp pα1**

| Verbindungen | Ki-Werte in [nM]^{a} | | | |
|---|---|---|---|---|
| | 5-HT1A | 5-HT2^{b} | α1^{c} | α1^{d} |
| **Beispiel 2** | 0,60 | 500 | 19 | 30 |
| **Beispiel 3** | 27 | 250 | --- | 73 |

| | | | | |
|---|---|---|---|---|
| ^{a} Durchschnittswerte aus 2-6 Einzelexperimenten durchgeführt mit Triplikaten ^{b} Ki-Wert bei gleichzeitiger Inkubation mit 10,uM Prazosin ^{c} Ki-Wert abgeleitet von der hochaffinen Bindungsstelle bei Markierung mit [³H]Ketanserin ^{d} Ki-Wert aus dem Kompetitionsexperiment mit dem α1-selektiven Radioliganden [³H]Prazosin | | | | |

## Patentansprüche

1. Verbindung der allgemeinen Formel (II) 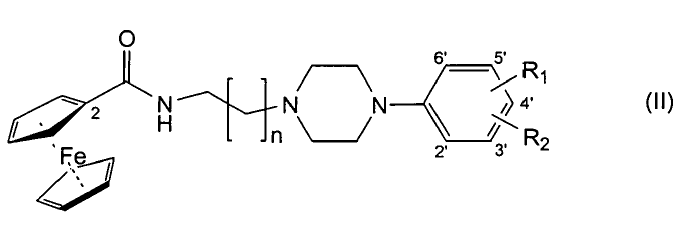 wobei
n = 1 - 4 sowie R₁ und R₂ einzeln oder auch zusammen für die Reste Wasserstoff, Hydroxy, Alkyloxy, Alkyl, Alkenyl, Alkinyl, Aryl, Halogen, Trifluormethyl, Acyl, Alkoxycarbonyl oder Cyano stehen.

2. Verbindung nach Anspruch 1, wobei R₁ und R₂ jeweils unabhängig voneinander ausgewählt sind aus Wasserstoff, Hydroxy, C1-C6-Alkyloxy, C1-C6-Alkyl, C2-C6-Alkenyl, C2-6-Alkinyl, Aryl, Fluor, Chlor, Brom, Trifluormethyl, C1-C6-Acyl, C1-C6-Alkoxycarbonyl und Cyano, worin die Gruppen C1-C6-Alkyl, C2-C6-Alkenyl, C2-C6-Alkinyl und Aryl unabhängig voneinander gegebenenfalls zusätzlich substituiert sind.

3. Verbindung nach Anspruch 2, ausgewählt aus:
N-4-(4-(2-Methoxyphenyl)piperazin-1-yl)butyl-2-ferrocenylcarbamid und
N-4-(4-(2,3-Dichlorphenyl)piperazin-1-yl)butyl-2-ferrocenylcarbamid.

4. Arzneimittel, das eine oder mehrere der Verbindungen gemäß einem der vorhergehenden Ansprüche enthält.

5. Arzneimittel nach Anspruch 4, das zusätzlich L-Dopa zur gleichzeitigen oder zeitlich aufeinanderfolgenden Verabreichung an den Patienten enthält.

6. Verwendung einer Verbindung nach einem der Ansprüche 1 bis 3 zur Herstellung eines Arzneimittels zur Therapie oder Prophylaxe von Kokain-, Alkohol-, Opiat- und Nikotinsucht; neurodegenerativen Störungen, insbesondere Morbus Parkinson; sexueller Dysfunktion; Depression oder Schizophrenie.

7. Verwendung einer Verbindung nach einem der Ansprüche 1 bis 3 zur Herstellung eines Arzneimittels zur Therapie oder Prophylaxe von Hyperprolaktinämie, Hyperprolaktinom, Glaucoma, kognitiven Störungen, Restless Leg-Syndrom, Hyperaktivitätssyndrom (ADHS), Parkinson-assoziierten Bewegungsstörungen, L-Dopainduzierten Störungen, Segawa-Syndrom, tardiven Bewegungsstörungen und zum Medikamenten-unterstützten Abstillen nach Schwangerschaften.

8. Verwendung nach Anspruch 7, wobei das Arzneimittel zur Therapie oder Prophylaxe von Segawa-Syndrom, spontaner Parkinson-assozüerter Dyskinesie oder Dystonie oder tardiver oder L-Dopa-induzierter Dyskinesie oder Dystonie vorgesehen ist.

9. Verwendung nach Anspruch 6, wobei das Arzneimittel zur Therapie von Schizophrenie vorgesehen ist.

10. Verfahren zur Herstellung einer Verbindung der allgemeinen Formel (II) wie oben definiert, umfassend das Umsetzen von Ferrocen-2-carbonsäure in aktivierter Form mit einer Verbindung der allgemeinen Formel (B'): 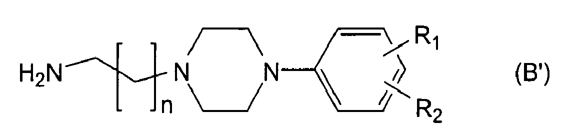 worin n, R₁ und R₂ wie in Formel (II) definiert sind.
